(19) Europäisches Patentamt / European Patent Office / Office européen des brevets

(11) **EP 4 185 184 B1**

(12) **EUROPEAN PATENT SPECIFICATION**

(45) Date of publication and mention
of the grant of the patent:
**10.07.2024  Bulletin 2024/28**

(21) Application number: **21749579.5**

(22) Date of filing: **23.07.2021**

(51) International Patent Classification (IPC):
*A61B 3/00* $^{(2006.01)}$        *A61B 3/113* $^{(2006.01)}$
*A61B 3/11* $^{(2006.01)}$        *G06V 40/16* $^{(2022.01)}$
*G06V 40/18* $^{(2022.01)}$

(52) Cooperative Patent Classification (CPC):
**A61B 3/113; A61B 3/111; G06V 40/171;
G06V 40/193**

(86) International application number:
**PCT/EP2021/070708**

(87) International publication number:
**WO 2022/018271 (27.01.2022 Gazette 2022/04)**

(54) **METHOD FOR DETERMINING A CORONAL POSITION OF AN EYE RELATIVE TO THE HEAD**

VERFAHREN ZUR BESTIMMUNG EINER KORONALEN POSITION EINES AUGES RELATIV ZUM KOPF

PROCÉDÉ DE DÉTERMINATION D'UNE POSITION CORONALE D'UN OEIL PAR RAPPORT À LA TÊTE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **24.07.2020  EP 20187649**

(43) Date of publication of application:
**31.05.2023  Bulletin 2023/22**

(73) Proprietor: **Universität Zürich
8006 Zürich (CH)**

(72) Inventors:
  • **WEBER, Konrad P.
    8006 Zürich (CH)**
  • **BOCKISCH, Christopher J.
    6300 Zug (CH)**
  • **POPOV, Todor
    8810 Horgen (CH)**
  • **FIERZ, Fabienne C.
    8706 Meilen (CH)**

(74) Representative: **Schulz Junghans
Patentanwälte PartGmbB
Großbeerenstraße 71
10963 Berlin (DE)**

(56) References cited:
**WO-A1-2020/110121      WO-A2-2020/235939
CN-A- 107 960 985      US-A1- 2012 010 533**

## Description

The invention relates to a computer-implemented method for determining a coronal position, particularly a distance of an eye relative to the coronal plane, with a handheld 3D-image capturing device, such as a smart phone, tablet or a handheld 3D-scanner.

Accurate and reproducible measurements of abnormal eyeball protrusion are important for diagnosing different causes of exophthalmos, as well as following patients with Grave's orbitopathy and retroorbital masses like optic nerve sheath meningioma.

The current clinical gold standard for measuring eyeball protrusion is the so-called Hertel exophthalmometer.

For a measurement of the coronal position of the eye, i.e. the protrusion of the eye, the Hertel exophthalmometer is placed on the lateral orbital rim of each eye by an examiner, using as little base as possible. Lines on a prism of the exophthalmometer are placed into position by moving the examiners viewpoint to determine the measurement. The measurement for the patient's right eye should be determined with the examiner's left eye and vice versa. The device measures the distance between the lateral orbital rim and the anterior position of the cornea. In general, measurements between the two eyes should be approximately equal.

Such a measurement procedure is not only time consuming, tedious, and prone to subjective reading errors, but always requires a professional examiner for conducting the measurement.

The use of handheld 3D-scanners for acquiring three-dimensional surfaces of the face for producing a custom-made face mask is known. However, a quantitative evaluation of a coronal position of the eye relative to the head is not readily accessible to a person, as no facial landmarks are determined.

Furthermore, Benz et al. [1], teach a method using a 3D-scanning device for determining a relative coronal position of the pupils to each other, wherein the test person has to wear specific lenses allowing the identification of the pupils by the method.

WO 2020/110121 A1 teaches a method and system for determining a person's point of gaze with a 3D-scanning device. Said document teaches, in particular, a method for determining a position, particularly a coronal position of an eye relative to a coronal plane of the head of a person with a handheld 3D-surface scanning device comprising the step of acquiring a digital data set comprising information about a three-dimensional surface and texture of at least a portion of the face of the person, the portion of the face comprising both eyes.

US 2012/010533 A1 discloses a method and system for determining a correct natural head position of a person based on the reference planes, including the coronal, sagittal, and transversal plane. The estimated planes may be used for profile analysis and treatment planning in which 3D facial analysis is the essence.

However, none of the methods, allow for a quantitative determination of the coronal position of eyes relative to the head.

Therefore, an objective of the invention is to provide an accurate, reliable and easy to use method to measure eyeball protrusion. The object is achieved by the method having the features of claim 1.

Advantageous embodiments are described in the dependent claims.

According to claim 1 a computer-implemented, particularly a smart-phone or tablet-implemented method for determining a coronal position, particularly comprising a distance of an eye relative to a plane parallel or identical to the coronal plane of the person, with a handheld 3D-surface-scanning device is disclosed. The method comprises at least the steps of:

- Acquiring a digital data set comprising information about a three-dimensional surface of at least a portion of the face of a person with the handheld 3D-scanning device, the portion of the face comprising both eyes and particularly the ears;
- Determining, particularly on the handheld 3D-scanning device or an external computer from the digital data set, particularly from a texture map comprised by the digital data set for each eye a three-dimensional position of the center of the pupil of the eye;
- Determining from the data set for each eye a three-dimensional rim position that corresponds to the anterior lateral orbital rim of the orbit;
- Determining a connecting line between the two rim positions, particularly wherein said connecting line is a straight line;
- Determining for each position of the center of the pupils a distance to the connecting line, wherein said distances correspond to the coronal position of each eye, particularly wherein the coronal position is a three-dimensional coordinate, particularly having the lateral coordinate components identical to the pupil positions, when the person looks straight forward;
- Particularly displaying the coronal positions particularly on a display.

The method can be implemented in a computer. The term "digital data set" particularly refers to a structured plurality of digital data, wherein said data is arranged on stored such that it can be retrieved, read and interpreted by a computer program. Data storage can be facilitated on a computer-readable, particularly non-transitory memory device.

The digital data set can be stored and handled in form of a computer-readable file on a computer.

The digital data set comprises information that is configured to represent at least a three-dimensional surface and a texture map, wherein the texture map comprises information on at least a brightness of a region, particularly wherein the texture map is representable as a gray-scale image or as a color image. Such information can be structured and stored in a variety of different data formats for representing a three-dimensional surface.

The three-dimensional surface can be represented within a framework of a coordinate system, such as a Cartesian coordinate system, having coordinates x, y and z.

In the context of the current specification the z-axis corresponds to the direction that is orthogonal to the coronal plane along which the eye position can be determined according to the invention. Thus, in anatomical terms, the z-axis extends orthogonal to the coronal plane. The term "coronal plane" is well-known in the art and relates to a vertical plane that divides the body of a person into ventral and dorsal sections. The coronal plane is sometimes also referred to as "frontal plane". In the context of the current specification the coronal plane may be further understood as the plane that extends through the head of the person and is fixed to the head of the person such that any pitch of the head results in a tilt of the coronal plane. Alternatively to the coronal plane, Reid's plane can be used to reference the coordinate system.

Therefore, the term "coronal plane" also includes Reid's plane in an alternative embodiment.

The coronal position is particularly a three-dimensional position relative to the coronal plane having the same lateral coordinates as the center positions of the pupils.

According to the invention, the coronal plane may comprise the connecting line between the rim positions.

Alternatively a spherical, cylindrical or another suitable coordinate system might be used for determining the coronal position instead.

In this context, the term "position" is to be understood with reference to the coordinate system. Generally however, the point of origin is of no particular importance for the method, as eventually the determined position of the center of the pupils and the rim positions are given in relation to each other so as to eliminate the specific point of origin.

The term "three-dimensional surface" particularly refers to a two-dimensional surface embedded in a three-dimensional space. Therefore, the surface is particularly a two-dimensional manifold embedded in three-dimensional space and has an orientation.

The three-dimensional surface can be characterized by means of a polygon mesh and/or in terms of a depth map.

The acquisition of the digital data is facilitated by means of the handheld 3D-image capturing device. Such device is for example a smart-phone, such as the iPhone 11 or the iPhone 12 (Apple Inc).

The acquisition is done with the handheld 3D-surface scanning device. Said device comprises for example a 3D-scanner, Lidar (light detection and ranging) scanner, a time of flight camera, and/or an image sensor cooperating with a projection device for projecting a spatial pattern on the surface so as to determine a three-dimensional shape of the surface and a texture map from an image captured by the image sensor. Thus, the 3D-surface scanning device is particularly an optical scanning device.

The 3D-surface scanning device particularly operates contactless to the surface to be scanned.

The term "3D" is an abbreviation for "three-dimensional". The 3D-surface-scanning device particularly scans the face point-, line- or area-wise. The latter corresponds to a parallel and/or simultaneous acquisition of the portion of the face.

The determination of the position of the center of the pupil of the eye can for example be achieved by a centroid fitting routine that is configured to determine a center of the pupil.

Regarding the step of determining the three-dimensional rim position that corresponds to the anterior lateral orbital rim of the eye it is noted that the anterior lateral orbital rim of the eye in the context of the current specification particularly refers to a portion of the face that extends over the zygomatic bone, where the rim of the eye's orbit has its largest recess. The anterior lateral orbital rim particularly corresponds to the position that is used as a support point for a conventional Hertel exophthalmometer.

The connecting line can be of virtual nature and is a straight line that is uniquely defined by the two rim positions, as it comprises the two rim positions.

The distance to the connecting line is particularly following conventional metrics, i.e. the distance for each eye is determined by means of the length of a straight line segment that connects the position of the center of the pupils with the connecting line, and is orthogonal to the connecting line. Therefore, each distance can therefore be associated to the straight line segment having an origin, a direction and a length, such that the coronal position of each can be unambiguously determined by means of said distance. In addition a point of origin of the coordinate system may be defined, wherein said point of origin is located at a fixed positon with respect ot he coronal plane or the Reid's plane. Particularly the point of origin is comprised in the coronal plane, more particularly the point of origin is arranged in the middle of the connecting line.

The distance, while being a relative measure, provides an accurate measure for the coronal position of the eyes, relative to the rim portion.

In this context the z-axis of the Cartesian coordinate system particularly extends along the line segments connecting the positions of the center of the pupils with the connecting line.

Thus, the determination of the coronal position can be determined independently of the orientation of the head of the person and for different head positions if necessary, e.g. looking down, looking up, or sideways when lying down. This is particularly due to the fact the coronal plane comprises the connecting line and therefore a coronal position can be established unambiguously.

According to an embodiment of the invention, the head position and orientation is determined from the digital data set and associated to the determined coronal position.

In order to make the result of the method accessible, the coronal positions might be displayed for example on a display. The coronal positions can be displayed graphically or numerically, i.e. in form of a value for each eye. Particularly, the coronal positions can be displayed in form of an overlay to the acquired three-dimensional surfaces.

According to another embodiment of the invention, the determined positions of the centers of the pupils, as well as the rim positions and the connecting line, can be displayed, particularly in an overlay view with the three-dimensional surface.

The method is configured to be executed on a software application, wherein the application is particularly executed on a mobile device, such as a smart phone or tablet, having a 3D-surface scanning device for executing the method according to the invention.

According to another embodiment of the invention, the coronal plane extends at a fixed angle to a plane close or identical to the radiological Reid's plane determined by the tragus of both ears and the lateral orbital rims.

For this purpose, a three-dimensional position of the tragus of each ear is determined from the digital data set, wherein a second connecting line is determined connecting the positions of the tragus of each ear, particularly wherein a head pitch angle of the coronal plane of the head relative to a horizontal plane is determined, such that deviations of the coronal position due to different head orientations can be accounted for and particularly wherein the coronal positon is corrected for the head pitch angle.

According to this embodiment, it is possible to use Reid's plane as an alternative system of reference.

According to this embodiment the data set therefore has to comprise information about a three-dimensional surface of at least a portion of the face of a person, the portion of the face comprising both ears.

The second connecting line is essentially parallel to the connecting line of the rim portion.

Therefore, this embodiment allows for a more robust and precise determination of the coronal plane of the head. Particularly, it allows for a more precise determination of the coronal positions as a pitch angle of the coronal plane of the head can be determined more reliably.

According to another embodiment of the invention, a three-dimensional position of the nasal bridge, particularly the notch of the nasal bridge is determined from the digital data set. The three-dimensional position of the nasal bridge provides an additional reference plane, based on which the protrusion of the eyes can be determined more accurately and more robust.

According to this embodiment a reference plane that extends parallel to the coronal plane or Reid's plane through the nasal bridge, particularly through the notch of the nasal bridge is determined, wherein the coronal positions of the eyes are also determined with respect to the additional reference plane such that the coronal positions of the eyes can be determined more accurately.

This embodiment allows for a more robust and precise determination of the protrusion of the eyes. Particularly, the three-dimensional position of the nasal bridge allows for a more precise determination of the coronal positions of both eyes.

According to the invention, for example by averaging the determined coronal positions a more robust estimate of the coronal positon is achieved.

According to another embodiment of the invention, the coronal position is determined relative to the positions of the tragus of each ear, relative to the second connecting line and/or relative to the nasal bridge.

The embodiment allows for a more robust and precise determination of the coronal position of the eyes, as not only one reference is used but at least one more reference for comparing the coronal positions with other facial features. The tragus and the nasal bridge are well defined and comparably easy to determine from an image, such that these additional "landmarks" in the face of a person provide an additional layer of robustness in terms of determination of the coronal position.

According to another embodiment of the invention, a direction of gaze is determined from the digital data set, wherein the angle of the determined plane of the iris is tilted in case the gaze direction is not straight forward, i.e. not orthogonal to the coronal plane of the head, such that the plane of the iris corresponds to the direction of gaze.

According to another embodiment of the invention, a computer-implemented pupil detection method is used for automatically determining the center positions of the eyes in the digital data set.

This embodiment allows for an automatic detection of the eyes and/or pupils and the automatic determination of the center positions of the pupils.

This embodiment provides a higher degree of automatization and increases the interoperability for different persons acquiring the digital data of the face, i.e. the method is less prone to subjective measurement errors.

The pupil detection method can comprise a Viola-Jones method [2] for eye detection paired with a method for determining the pupil's centers on the detected eyes.

According to another embodiment of the invention, a computer-implemented rim detection method is used for automatically determining the rim positions.

This embodiment provides an even higher degree of automatization and increases the interoperability for different persons acquiring the digital data of the face, i.e. the method is less prone to subjective measurement errors.

The rim detection method can make use of the acquired three-dimensional surface as well as additional information such as color or texture information or additional marker information acquired in the digital data set.

According to another embodiment of the invention, the information about the three-dimensional surface is represented in form of a face-vertex polygon mesh in the digital data set.

The face-vertex polygon mesh is well suited for representing the three-dimensional surface of the face with sufficient accuracy and thus balancing spatial resolution and data compression allowing economical use of memory space and reducing the computational load.

There are a variety of file formats designed for storing face-vertex polygon meshes that can be used by the method according to the invention.

According to an embodiment of the invention, the 3D-surface scanning device can be configured to generate and provide the method said face-vertex polygon mesh and/or a file having the polygon mesh stored in a suitable file format.

According to another embodiment of the invention, the digital data set comprises information about a color and/or texture information of the three-dimensional surface, wherein said information is associated to the three-dimensional surface.

The color information can be a gray-scale information only, for example representing a brightness, an intensity and/or a luminance. Alternatively or additionally, the color information can comprise an RGB, CMYK or an alternative representation of the color space.

According to this embodiment the 3D-surface-scanning device is configured to record color information together with the spatial information of the surface. The color information gives rise to a texture map that can be represented as a two-dimensional color image of the face. Alternatively, the three-dimensional surface can be overlaid with the color /texture map such that a three-dimensional representation of the face is facilitated.

The color information allows for an additional parameter, namely the color information, to be exploited by the method according to the invention, particularly by the pupil detection method and/or the rim detection method, which provides a higher degree of robustness and accuracy.

Associating the color information with the polygon mesh allows for the following embodiment.

According to another embodiment of the invention, the information about the color is associated to the polygon mesh in form of a texture map mapping the information about the color to the faces and vertices of the polygon mesh.

A texture map particularly comprises the color information and/or a brightness or a luminance information. Texture mapping of a vertex-face polygon mesh can be achieved rapidly and allows for fast processing of the digital data set.

According to this embodiment each face has at least one associated color value indicative for the color, and particularly also an associated brightness and/or a luminance value.

From the color information automatic determination of specific portions, e.g. the eyes or the rim portions, of the face can be performed in a more reliable fashion.

According to another embodiment of the invention, the anterior lateral orbital rim is labelled with a marker having a color different to a skin tone of the person whose face is acquired by the 3D-surface scanning device, before the digital data set is acquired, particularly wherein the rim positions are determined by the rim detection method by searching for the color of the marker in in the digital data set.

This embodiment allows for a more robust estimation of the rim positions and thus for a more accurate determination of coronal positions.

Alternatively, a trained machine learning method can be used as the rim detection method.

According to another embodiment of the invention, in a first step of the method, the center positions of the pupils are determined and subsequently the rim positions are determined by the rim detection method, wherein the rim detection method limits its search for rim positions to portions of the face that are located laterally from the determined center positions.

This embodiment allows for a more robust and faster determination of the rim positions.

According to another embodiment of the invention, the pupil detection method executes the following steps:

- Determining a portion of the digital data set that comprises the eyes, particularly using a Viola-Jones eye-detection method,

- Filtering the digital data representing said portion with a low pass filter, particularly with an isotropic low pass filter,

- Determining the center position of the pupils from the filtered digital data, for example with a centroid fitting method.

The filtering step allows for removal of unwanted details such as eye lashes and noise, and thus enhancing the accuracy of the determination of the center positions.

When the filter is an isotropic low pass filter, the shape features of the pupils are not distorted along a specific direction.

According to another embodiment of the invention, the handheld 3D-surface-scanning device is comprised in a mobile computerized device, such as a smart phone or tablet.

A smart phone that for example comprises such a 3D-surface scanning devices is for example the iPhone 11 from Apple Inc. or the iPhone 12. The 3D-surface-scanning device is the so-called True-Depth camera.

This embodiment allows for convenient execution of the method according to the invention with a mobile computerized device.

The mobile device can be programmed to execute the method according to the invention, or to perform at least some of the method steps.

According to another embodiment of the invention, the handheld 3D-surface-scanning device determines the three-dimensional surface of the face by projecting a plurality of optical markers on the face of the person, wherein the handheld 3D-surface-scanning device further captures the information about the color such that the optical markers can be retrieved by the method.

From the acquired images comprising the markers, a depth map can be generated by the scanning device, wherein said depth map can be converted to the polygon mesh for further analysis.

It is noted that the markers are particularly projected in the invisible infrared spectral region so that the person whose face is scanned is not distracted by the projected markers and so that the recorded color information relating to the visible spectrum is not distorted by the markers.

According to another embodiment of the invention, the coronal positions are determined repeatedly on different days, wherein information about a difference between the coronal positions that have been determined on different days is displayed.

This embodiment allows monitoring progression of eye protrusion in clinical or other settings.

The problem according to the invention is furthermore solved by a computer program.

According to this aspect of the invention, the computer program comprises computer program code that, when executed on a computer, such as a smartphone or a tablet, causes the computer to execute at least the following computer-implemented steps of the method according to the invention:

- Reading the digital data set from a memory of the computer;

- Determining the center positions of the pupils with the pupil detection method;

- Determining the rim positions with the rim detection method;

- Determining a connecting line between the two rim positions;

- Determining for each center of the pupils a distance to the connecting line, said distances corresponding to the coronal position of each eye relative to the head;

- Displaying information about the determined coronal positions on a display.

The computer program allows for precise and accurate determination of the coronal position of the eyes in relation to the head.

The computer is particularly connected to the 3D-surface scanning device or comprises the 3D-surface scanning device.

According to another embodiment the computer program further executes the steps of:

- Comparing the determined coronal positions to coronal positions that have been determined on previous days from the same person,

- Displaying a graphical representation providing information about a deviation of the determined coronal positions and the coronal positions that have been determined on previous days.

This embodiment allows for tracking of the coronal positions over time.

According to another embodiment of the invention, the computer program causes the handheld 3D-surface-scannig device to acquire the digital data set and store the acquired data set on the memory device of the computer.

According to an embodiment, the computer program is executed on the handheld 3D-surface scanning device, wherein said device is a computerized device, particularly a mobile device such as a smart phone or tablet.

According to another embodiment of the invention, the computer program is executed on the mobile device, and is configured to record and evaluate the acquired digital data and to send the coronal positions and/or the digital data to a second computer for storing the results and/or for displaying the coronal positions.

According to another embodiment of the invention, the computer program is furthermore, configured to display the coronal position and/or the acquired digital data on a display of the mobile device.

According to another embodiment of the invention, a three-dimensional position of the tragus of each ear is determined from the digital data set by the computer program, wherein a second connecting line is determined connecting the positions of the tragus of each ear. The pitch angle of the coronal plane of the head is then defined with reference to a horizontal plane close or identical to the radiological Reid's plane determined by the tragus of both ears and the lateral orbital rims, such that deviations of the coronal position due to different head orientations can be accounted for.

According to another embodiment of the invention, a three-dimensional position of the bridge of the nose, particularly the notch of the bridge of the nose between forehead and nose is determined from the digital data set by the compute program, such that deviations of the coronal position of the eyes can also be referenced to the nose bridge in addition to the orbital rims.

According to another embodiment of the invention, the coronal position is determined relative to the positions of the tragus of each ear, relative to the second connecting line and/or relative to the nasal bridge by the computer program.

The term "computerized device" or computerized system or a similar term denotes an apparatus comprising one or more processors operable or operating according to one or more programs, particularly a mobile device such as a mobile phone or tablet.

The term "mobile device" particularly refers to a small computer device, particularly small enough to hold and operate in the hand and having an operating system capable of running mobile apps - software applications designed to run on mobile devices. A mobile device is therefore a computerized device that is portable and particularly weights less than 500 g.

A mobile device, such as a mobile phone, a smart phone, a smart watch, or a tablet computer, particularly comprises at least one processor, the so-called CPU (central processing unit). Furthermore, a mobile device particularly comprises means for cellular network connectivity for connecting to a mobile network, such as for example GSM (Global System for Mobile Communications), 3G, 4G or 5G, CDMA (Code Division Multiple Access), CDMA2000, UTMS (Universal Mobile Telecommunications System), or LTE (Long Term Evolution).

The mobile device comprises a display screen with a numeric or an alphanumeric keyboard or a touchscreen configured to provide a virtual keyboard and buttons (icons) on-screen. The mobile device is particularly configured to connect to the Internet and interconnect with other computerized devices via Wi-Fi, Bluetooth or near field communication (NFC). Integrated cameras, depth cameras, optical 3D-scanning devices, mobile phone and GPS capabilities are common.

The mobile device is particularly configured to provide a time reference for apps and data, wherein said time reference is provided by a time reference system such as an internal clock or timer of the mobile device or by external signals that are for example received by the internet, the mobile network, or GPS. The time reference can be adjusted for different time zones.

The terms 'processor' or 'computer', or system thereof, are used herein as ordinary context of the art, such as a general purpose processor or a micro-processor, RISC processor, or DSP, possibly comprising additional elements such as memory or communication ports. Optionally or additionally, the terms 'processor' or 'computer' or derivatives thereof denote an apparatus that is capable of carrying out a provided or an incorporated program and/or is capable of controlling and/or accessing data storage apparatus and/or other apparatus such as input and output ports. The terms 'processor' or 'computer' denote also a plurality of processors or computers connected, and/or linked and/or otherwise communicating, possibly sharing one or more other resources such as a memory.

The terms 'application', 'app', 'program', or 'computer program', or 'computer program code' may be used interchangeably according to the context thereof, and denote one or more instructions or directives or circuitry for performing a sequence of operations that generally represent an algorithm and/or other process or method. The program is stored in or on a medium such as RAM, ROM, SSD or hard disk, or embedded in a circuitry accessible and executable by an apparatus such as a processor or other circuitry.

The processor and program may constitute the same apparatus, at least partially, such as an array of electronic gates, such as FPGA or ASIC, designed to perform a programmed sequence of operations, optionally comprising or linked with a processor or other circuitry.

Particularly, exemplary embodiments are described below in conjunction with the Figures. The Figures are appended to the claims and are accompanied by text explaining individual features of the shown embodiments and aspects of the present invention. Each individual feature shown in the Figures and/or mentioned in said text of the Figures may be incorporated (also in an isolated fashion) into a claim relating to the method or the computer program according to the present invention.

Fig. 1    shows the anterior lateral orbital rim and the rim position;

Fig. 2    shows a flow diagram of an embodiment the method according to the invention; and

Fig. 3    illustrates some aspects of the method according to the invention.

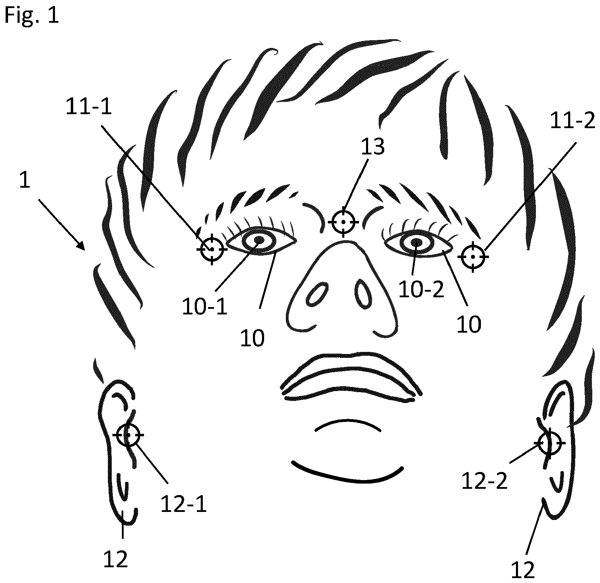

Fig. 1 shows a schematic of a head 1 with the anterior lateral orbital rim of the eyes 10 that correspond to the rim positions 11-1, 11-2 that are to be determined. It is noted that the rim position is the corresponding position on the face, i.e. with skin and soft tissue on the skull bones.

Moreover, the tragus 12-1, 12-2 of both ears 12 as well as the nasal bridge 13 is shown.

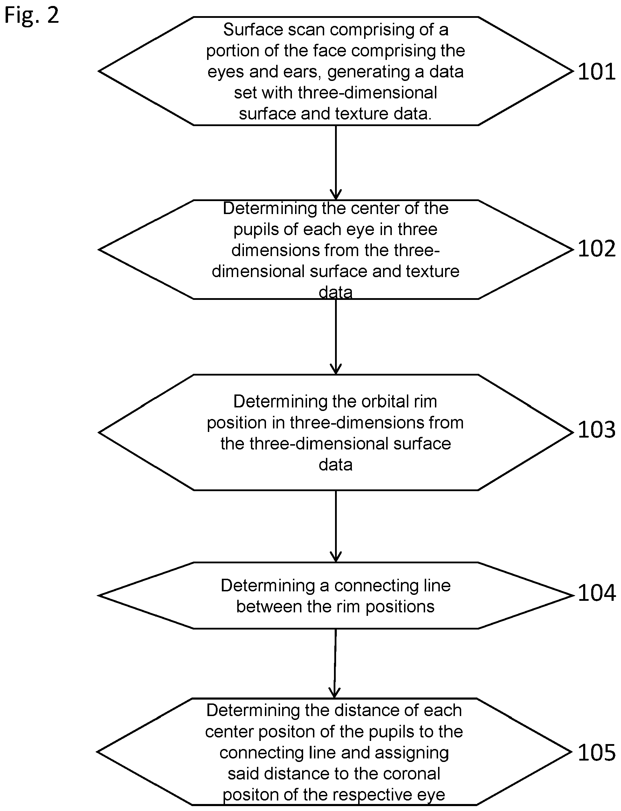

Fig. 2 shows a schematic flow diagram of an embodiment of the invention. In a first step 101, a three-dimensional surface scan of a region of the face is acquired, wherein said region comprises both eyes and potentially the ears. The surface scan is performed with a handheld optical scanning device. The scanning device can be a smartphone or tablet with a camera that is configured for such three-dimensional surface scans.

For this purpose for example a conventional iPhone 11 smartphone from Apple Inc. can be used. Said smartphone comprise a camera equipped for three-dimensional imaging.

In order to generate the three-dimensional information, the smartphone might project an infrared light pattern on the face of the person from which the three-dimensional scan is to be recorded.

Combined with at least one recorded two-dimensional image of the face the smartphone calculates a depth map for the face. The depth map essentially assigns three-dimensional information to the at least one image that has been recorded.

The depth map and the at least one image are processed further in such a way that the three-dimensional surface comprising the face is represented by a polygon mesh, particularly a vertex-face mesh with an associated texture map comprising the color or gray-scale information from the at least one image, such that a complete three-dimensional color representation of the face can be provided e.g. for display on a screen.

The data format that is used for storing the measurements with the scanning device is a 3D graphics file format, for example a wavefront OBJ file. Said file contains the digital data associated to the scanned three-dimensional surface of the subject's head, that describes the face as a collection of polygon faces (the thee-dimensional vertex locations, as well which vertices are grouped together to form a face), and an associated texture map representing an image of the face which contains the color information.

The texture map wraps on the surface of the face according to the polygonal mesh.

In a next step 102, the texture map, i.e. the color information is used to determine the eyes of the subject person. This can be done by conventional methods, such as Viola-Jones eye detection method (cf. Viola and Jones [2]) that provides a limited region within which an image of the eyes of the subject person is located. Other detection methods might equally suitable for this task. In a second step the center positions of the eyes are identified within the regions. While this process is described as a two-step process it is possible to use a computer-implemented pupil detection method that is configured to automatically determining the center positions of the eyes in the digital data set, particularly without first detecting the eyes. Such method is for example an artificial neuronal network that is trained for this task.

Once the pupil centers are determined in the texture map, it is straight forwards to map said positions to the polygon mesh representing the three-dimensional surface. From this mapping, a three-dimensional coordinate for each pupil center is obtained.

While the specific absolute positions with respect to the origin is not important for further processing steps, it can be assumed that the origin is located in a center of mass of the three-dimensional surface.

In step 103 the texture map is used to aid the detection of the anterior lateral orbital rims or alternatively the nasal bridge. The rim positions corresponding to the anterior lateral orbital rims are sometimes not easy to detect, but can be found by a person e.g. by touching. For the purpose of robust detection the rim positions can be marked with ink (with a non-skin tone color, such as green) from a pen, such that are readily visible on the texture map.

Alternatively a trained machine learning method, for example an artificial neural network can be used to identify the rim positions in the texture map. Moreover, it is possible to also use the information form the three-dimensional surface for detection of the rim positions.

When the rim positions are determined, a virtual straight connecting line is determined, as depicted in step 104 of Fig. 2, said connecting line comprising the rim positions.

In a subsequent step, step 105, for each center position of the pupil a distance to said connecting line is determined. A suitable distance metric is described in the following.

A line segment connecting the center position of the pupil and the connecting line is determined such that said line segment is oriented orthogonal to the connecting line. The distance is then given by the length of the line segment. Thus, according to an embodiment of the invention, for each center position such a line segment is determined and for each line segment the corresponding length is calculated.

The length of each line segment in turn is assigned to the coronal position of the eye of the subject person. The coronal direction is then particularly defined by the direction of the respective line segment.

In mathematical terms the connecting line a can be expressed as follows:

$$a = RP1 - RP2$$

where RP1 is the rim position of the left eye orbit, RP2 is the rim position of the right eye orbit, and a is the connecting line of the rim positions.

$$b1 = PUP1 - RP1; b2 = PUP2\text{-}RP2;$$

b1 is a line segment extending from the rim position RP1 of the left eye to the center position PUP1 of the pupil of the left eye, wherein b1 is a line segment extending from the rim position RP2 of the right eye to the center position PUP2 of the pupil of the right eye.

The distance d1 of the left center position to the connecting line a is therefore given by:

$$d1 = \frac{|(a \times b1)|}{|a|} = \frac{\sqrt{\sum (a \times b1)^2}}{\sqrt{\sum a^2}}$$

wherein (a × b1) indicates the cross-product between a and b1, and the vertical lines denote vector length. In a similar fashion the distance d2 of the right center position to the connecting line a is given by:

$$d2 = \frac{|(a \times b2)|}{|a|} = \frac{\sqrt{\sum (a \times b2)^2}}{\sqrt{\sum a^2}}$$

The determined coronal positions are then displayed to a person. This can be done in various ways. For example the value of the coronal position can be displayed and/or a graphical representation of said position particularly in form of an overlay with a display of the three-dimensional surface and/or the texture map.

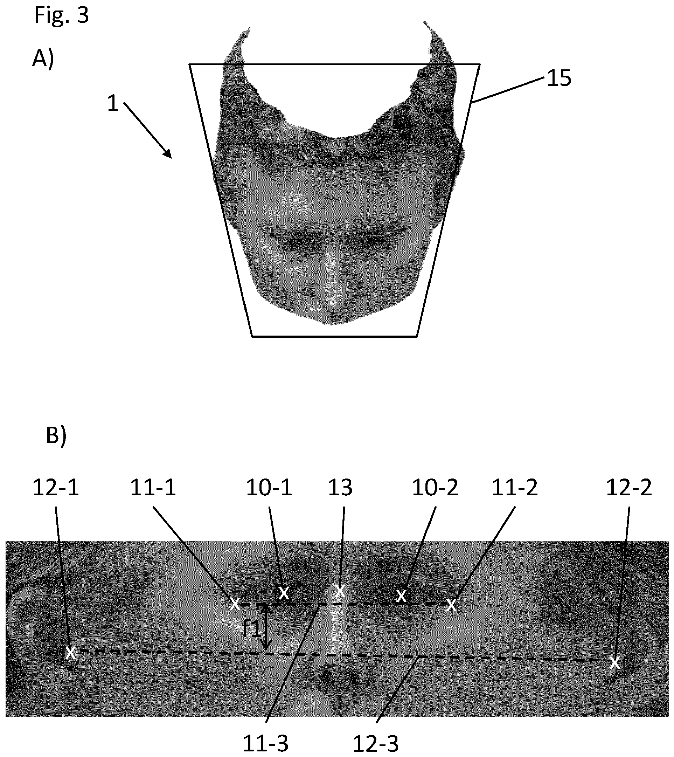

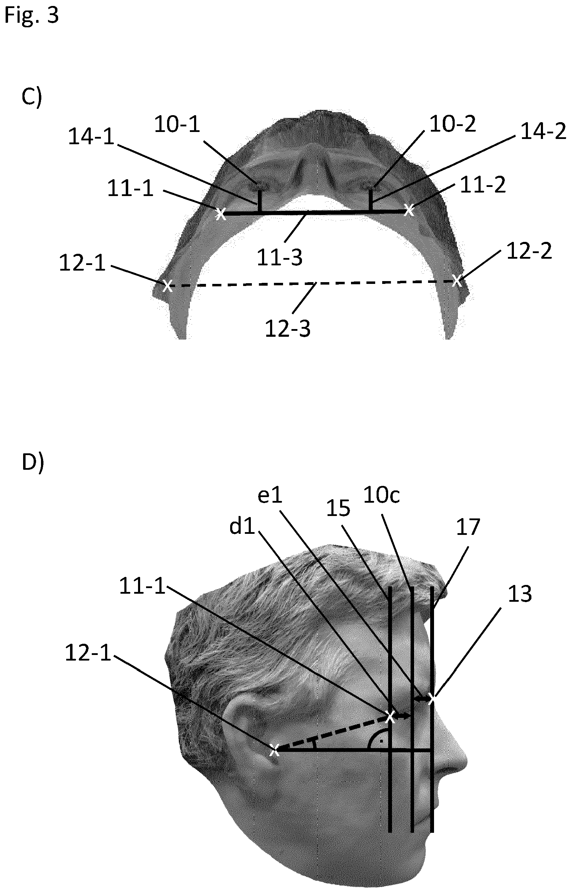

In Fig. 3, panel A, the three-dimensional recording of a face of a subject person is shown. The three-dimensional surface is overlaid with the texture map comprising the color information (in the example shown it is gray scale information only).

In this example the rim positions 11-1, 11-2 have been marked with a green marker (shown as an "x"), such that they are readily identified in the texture map. This is illustrated in panel B of Fig. 3.

In Fig. 3, panel B, both, the rim positions 11-1, 11-2 as well as the determined pupil center positions 10-1, 10-2 (also marked with an "x") are shown.

In panel C of Fig. 3, the connecting line a (11-3) between the rim positions RP1, RP2 (11-1, 11-2) is shown in a bottom-up view of the three dimensional surface. The line segments 14-1, 14-2 connecting the center positions PUP1, PUP2 (10-1, 10-2) of the pupils with said connecting line 11-3 are indicative of the distance d1, d2 and thus the coronal position of the eyes relative to the head and the coronal plane 15 (cf. Fig. 3A). The distance d1 and d2 might be assigned to the coronal position with regard to the z-axis and the pupil positions might be assigned as the x- and y- coordinate of the coronal position.

In Figure 3, panel D, a side view of the data set is shown. Here, the coronal plane 15 as well as the additional reference plane 17 can be seen. The additional reference plane extends parallel to the coronal plane through the notch of the nasal bridge 13 and serves as an additional reference for determining the coronal positions of the eyes. The plane 10c in which the coronal position of the left eye 10-1 is located is indicated by 10c. The distances d1, e1 to the coronal plane 15 and to the additional reference plane 17 provide two independent measures for the coronal positons of the eyes such that a more robust estimation of the coronal posiotn can be achived.

Further, the position 12-1 of the left tragus is shown that can be used to estimate a pitch of the head and thus a pitch of the coronal plane. Therefore, any pitch of the head may be accounted for in order to grant a determination of the coronal positions independent of the pitch of the head.

For example for illustrative purpose, when the head assumes an angle, the connecting line 11-3 and the second connecting line 12-3 assume a different projected distance f1 that is indicative for a pitch of the head.

In general, as the connecting line 11-3 and the second connecting line 12-3 are extend in three dimensions a pitch of the head can be determined in any event, also without using the projected distance f1 as will be recognized by the person skilled in the art.

References

[1] Benz et al, " Optical 3d-metrology for medical applications", Proceedings of ICMP 2005 and BMT, Berlin. 2005: 64-65.

[2] Viola and Jones, "Rapid object detection using a boosted cascade of simple features", International Journal of Computer Vision, 2001.

**Claims**

1. A method for determining a position, particularly a coronal position of an eye (10) relative to a coronal plane (15) of the head (10) of a person, with a handheld 3D-surface scanning device, wherein the method comprises the steps of:

   - Acquiring a digital data set comprising information about a three-dimensional surface and texture of at least a portion of the face of the person, the portion of the face comprising both eyes (10);
   - Determining from the data set for each eye (10) a three-dimensional center position (10-1, 10-2) of the center of the pupil of the eye (10);
   - Determining from the digital data set for each eye (10) a three-dimensional rim position (11-1, 11-2) that corresponds to the anterior lateral orbital rim of the eye (10);
   - Determining a connecting line (11-3) between the two rim positions (11-1, 11-2);
   - Determining for each three-dimensional center position of the centers of the pupils (10-1, 10-2) a distance (14-1, 14-2) to the connecting line (11-3), wherein each distance (14-1, 14-2) corresponds to a coronal position of an eye (10) relative to the coronal plane (15) of the head (1).

2. The method according to claim 1, wherein a three-dimensional position (12-1, 12-2) of the tragus of each ear (10) is determined from the digital data set, wherein a second connecting line (12-3) is determined that connects the positions (12-1, 12-2) of the tragus of each ear (12), particularly wherein a head pitch angle of the coronal plane (15) of the head relative to a horizontal plane is determined, such that deviations of the coronal position due to different head orientations are compensated for.

3. The method according to claim 1 or 2, wherein a three-dimensional position of the nasal bridge (13), particularly the notch of the nasal bridge is determined from the digital data set, wherein an additional reference plane (17) that extends parallel to the coronal plane (15) through the nasal bridge, particularly through the notch of the nasal bridge, is determined, wherein the coronal positions of the eyes (10) are also determined with respect to the additional reference plane (17) such that the coronal positions of the eyes (10) are determined more accurately.

4. The method according to claim 2 or 3, wherein the coronal position is determined relative to the positions (12-1, 12-2) of the tragus of each ear (12), relative to the second connecting line and/or relative to the nasal bridge (13).

5. The method according to any of the preceding claims, wherein a computer-implemented pupil detection method is used for automatically determining the center positions (10-1, 10-2) of the eyes (10) in the digital data set.

6. The method according to any of the preceding claims, wherein a computer-implemented rim detection method is used for automatically determining the rim positions (11-1, 11-2).

7. The method according to any of the preceding claims, wherein the information about the three-dimensional surface is represented in form of a face-vertex polygon mesh in the digital data set.

8. The method according to any of the preceding claims, wherein the digital data set comprises information about a color or a gray scale texture of the three-dimensional surface, wherein said information is associated to the three-dimensional surface.

9. The method according to claim 7 and 8, wherein the information about the color or gray scale is associated to the polygon mesh in form of a texture map mapping the information about the color or the gray scale to the faces and vertices of the polygon mesh.

10. The method according to any of the claims 6 to 9, wherein the anterior lateral orbital rim is labelled with a marker having a color different to a skin tone of the person before the digital data set is acquired, wherein the rim positions (11-1, 11-2) are determined by the rim detection method by searching for the color of the marker in in the digital data set.

11. The method according to any of the claims 6 to 10, wherein first the center positions (10-1, 10-2) of the pupils are determined and then rim positions (11-1, 11-2) are determined by the rim detection method, wherein the rim detection method limits its search for rim positions to portions of the face that are located laterally from the determined center positions (10-1, 10-2) of the pupils.

12. The method according to any of the preceding claims, wherein the pupil detection method executes the following steps:

   - Determining a portion of the digital data set that comprises the eyes (10),
   - Filtering the digital data representing said portion with a low-low pass filter,
   - Determining the center position (10-1, 10-2) of the pupils from the filtered digital data.

13. The method according to any of the preceding claims, wherein the handheld 3D-surface-scanning device is comprised in a mobile device, such as a smart phone or tablet.

14. A computer program comprising computer-program code, that when executed on a computer, causes the computer to execute the at least the following computer-implemented steps of the method according to any of the preceding claims:

   - Reading from a memory of the computer the digital data set,
   - Determining the center positions (10-1, 10-2) of the pupils with the pupil detection method,
   - Determining the rim positions (11-1, 11-2) with the rim detection method,
   - Determining a connecting line (11-3) between the two rim positions (11-1, 11-2),
   - Determining for each center position (10-1, 10-2) of the pupils a distance to the connecting line (11-3), said distances corresponding to the coronal position of each eye (10) relative to the coronal plane (15) of the head;
   - Displaying information about the determined coronal positions on a display.

15. Computer program according to claim 14, wherein the computer program further executes the steps of:

   - Comparing the determined coronal positions to coronal positions that have been determined on previous days from the same person,
   - Displaying a graphical representation providing information about a deviation of the determined coronal positions and the coronal positions that have been determined on previous days.

**Patentansprüche**

1.  Verfahren zum Bestimmen einer Position, insbesondere einer koronalen Position eines Auges (10) relativ zu einer koronalen Ebene (15) des Kopfes (10) einer Person, mit einer handgeführten 3D-Oberflächenabtastvorrichtung, wobei das Verfahren die folgenden Schritte umfasst:

    - Erfassen eines digitalen Datensatzes, der Informationen über eine dreidimensionale Oberfläche und Textur von mindestens einem Teil des Gesichts der Person umfasst, wobei der Teil des Gesichts beide Augen (10) umfasst;
    - Bestimmen einer dreidimensionalen Mittelposition (10-1, 10-2) der Mitte der Pupille des Auges (10) aus dem Datensatz für jedes Auge (10);
    - Ermitteln einer dreidimensionalen Randposition (11-1, 11-2) aus dem digitalen Datensatz für jedes Auge (10), die dem vorderen lateralen Orbitalrand des Auges (10) entspricht;
    - Ermitteln einer Verbindungslinie (11-3) zwischen den beiden Randpositionen (11-1, 11-2);
    - Bestimmen eines Abstands (14-1, 14-2) zu der Verbindungslinie (11-3) für jede dreidimensionale Mittelposition der Zentren der Pupillen (10-1, 10-2), wobei jeder Abstand (14-1, 14-2) einer koronalen Position eines Auges (10) relativ zu der koronalen Ebene (15) des Kopfes (1) entspricht.

2.  Verfahren nach Anspruch 1, wobei aus dem digitalen Datensatz eine dreidimensionale Position (12-1, 12-2) des Tragus eines jeden Ohres (10) ermittelt wird, wobei eine zweite Verbindungslinie (12-3) ermittelt wird, die die Positionen (12-1, 12-2) des Tragus eines jeden Ohres (12) verbindet, insbesondere wobei ein Kopfnickwinkel der Koronalebene (15) des Kopfes relativ zu einer horizontalen Ebene ermittelt wird, so dass Abweichungen der Koronalposition aufgrund unterschiedlicher Kopforientierungen kompensiert werden.

3.  Verfahren nach Anspruch 1 oder 2, wobei aus dem digitalen Datensatz eine dreidimensionale Position des Nasenstegs (13), insbesondere der Kerbe des Nasenstegs, ermittelt wird, wobei eine zusätzliche Referenzebene (17), die parallel zur Koronalebene (15) durch den Nasensteg, insbesondere durch die Kerbe des Nasenstegs, verläuft, ermittelt wird, wobei die koronalen Positionen der Augen (10) auch in Bezug auf die zusätzliche Referenzebene (17) ermittelt werden, so dass die koronalen Positionen der Augen (10) genauer bestimmt werden.

4.  Verfahren nach Anspruch 2 oder 3, wobei die koronale Position relativ zu den Positionen (12-1, 12-2) des Tragus eines jeden Ohres (12), relativ zur zweiten Verbindungslinie und/oder relativ zum Nasenrücken (13) bestimmt wird.

5.  Verfahren nach einem der vorhergehenden Ansprüche, wobei ein computerimplementiertes Pupillenerkennungsverfahren zur automatischen Bestimmung der Mittelpositionen (10-1, 10-2) der Augen (10) im digitalen Datensatz verwendet wird.

6.  Verfahren nach einem der vorhergehenden Ansprüche, bei dem ein computerimplementiertes Randdetektionsverfahren zur automatischen Bestimmung der Randpositionen (11-1, 11-2) verwendet wird.

7.  Verfahren nach einem der vorhergehenden Ansprüche, wobei die Information über die dreidimensionale Oberfläche in Form eines Flächen-Vertex-Polygonnetzes in dem digitalen Datensatz dargestellt wird.

8.  Verfahren nach einem der vorhergehenden Ansprüche, wobei der digitale Datensatz Informationen über eine Farbe oder eine Graustufentextur der dreidimensionalen Oberfläche umfasst, wobei diese Informationen der dreidimensionalen Oberfläche zugeordnet sind.

9.  Verfahren nach Anspruch 7 und 8, wobei die Informationen über die Farbe oder die Graussstufen dem Polygonnetz in Form einer Texturkarte zugeordnet werden, die die Informationen über die Farbe oder die Graussstufen auf die Flächen und Scheitelpunkte des Polygonnetzes abbildet.

10. Verfahren nach einem der Ansprüche 6 bis 9, wobei der vordere laterale Orbitalrand mit einer Markierung gekennzeichnet wird, die eine Farbe hat, die sich von einem Hautton der Person unterscheidet, bevor der digitale Datensatz erfasst wird, wobei die Randpositionen (11-1, 11-2) durch das Randerfassungsverfahren bestimmt werden, indem nach der Farbe der Markierung in dem digitalen Datensatz gesucht wird.

11. Verfahren nach einem der Ansprüche 6 bis 10, wobei zunächst die Mittenpositionen (10-1, 10-2) der Pupillen bestimmt werden und dann die Randpositionen (11-1, 11-2) durch das Randerkennungsverfahren bestimmt werden, wobei das Randerkennungsverfahren seine Suche nach Randpositionen auf Teile des Gesichts beschränkt, die seitlich von den bestimmten Mittenpositionen (10-1, 10-2) der Pupillen liegen.

12. Verfahren nach einem der vorhergehenden Ansprüche, wobei das Pupillenerkennungsverfahren die folgenden Schritte ausführt:

- Ermitteln eines Teils des digitalen Datensatzes, der die Augen (10) umfasst,
- Filtern der digitalen Daten, die diesen Teil darstellen, mit einem Tiefpassfilter,
- Ermitteln der Mittelposition (10-1, 10-2) der Pupillen aus den gefilterten digitalen Daten.

13. Verfahren nach einem der vorhergehenden Ansprüche, wobei die handgeführte 3D-Oberflächenabtastvorrichtung in einem mobilen Gerät, wie einem Smartphone oder Tablet, enthalten ist.

14. Computerprogramm, das einen Computerprogrammcode umfasst, der, wenn er auf einem Computer ausgeführt wird, den Computer veranlasst, mindestens die folgenden computerimplementierten Schritte des Verfahrens nach einem der vorangehenden Ansprüche auszuführen:

   - Auslesen des digitalen Datensatzes aus einem Speicher des Computers,
   - Ermitteln der Mittelpositionen (10-1, 10-2) der Pupillen mit dem Pupillenerkennungsverfahren,
   - Ermitteln der Randpositionen (11-1, 11-2) mit dem Randdetektionsverfahren,
   - Ermitteln einer Verbindungslinie (11-3) zwischen den beiden Randpositionen (11-1, 11-2),
   - Bestimmen eines Abstandes zur Verbindungslinie (11-3) für jede Mittelposition (10-1, 10-2) der Pupillen, wobei die Abstände der koronalen Position jedes Auges (10) relativ zur koronalen Ebene (15) des Kopfes entsprechen;
   - Anzeige von Informationen über die ermittelten Koronalpositionen auf einem Display.

15. Computerprogramm nach Anspruch 14, wobei das Computerprogramm ferner die folgenden Schritte ausführt:

   - Vergleichen der ermittelten Koronalpositionen mit Koronalpositionen, die an vorangegangenen Tagen bei derselben Person ermittelt wurden,
   - Anzeigen einer grafischen Darstellung, die Informationen über eine Abweichung der ermittelten Koronalpositionen und der an vorangegangenen Tagen ermittelten Koronalpositionen liefert.

**Revendications**

1. Procédé de détermination d'une position, notamment d'une position coronale d'un oeil (10) par rapport à un plan coronal (15) de la tête (10) d'une personne, avec un dispositif de balayage de surface tridimensionnelle portable, dans lequel le procédé comprend les étapes consistant à :

   - acquérir un ensemble de données numériques comprenant des informations concernant une surface et texture tridimensionnelles d'au moins une portion du visage de la personne, la portion du visage comprenant les deux yeux (10) ;
   - déterminer à partir de l'ensemble de données pour chaque oeil (10) une position de centre tridimensionnelle (10-1, 10-2) du centre de la pupille de l'oeil (10) ;
   - déterminer à partir de l'ensemble de données numériques pour chaque oeil (10) une position de bord tridimensionnelle (11-1, 11-2) qui correspond au bord orbital latéral antérieur de l'oeil (10) ;
   - déterminer une ligne de connexion (11-3) entre les deux positions de bord (11-1, 11-2) ;
   - déterminer pour chaque position de centre tridimensionnelle des centres des pupilles (10-1, 10-2) une distance (14-1, 14-2) à la ligne de connexion (11-3), dans lequel chaque distance (14-1, 14-2) correspond à une position coronale d'un oeil (10) par rapport au plan coronal (15) de la tête (1).

2. Procédé selon la revendication 1, dans lequel une position tridimensionnelle (12-1, 12-2) du tragus de chaque oreille (10) est déterminée à partir de l'ensemble de données numériques, dans lequel une seconde ligne de connexion (12-3) est déterminée, laquelle connecte les positions (12-1, 12-2) du tragus de chaque oreille (12), notamment dans lequel un angle d'inclinaison de tête du plan coronal (15) de la tête par rapport à un plan horizontal est déterminé de sorte que des écarts de la position coronale en raison de différentes orientations de tête sont compensés.

3. Procédé selon la revendication 1 ou 2, dans lequel une position tridimensionnelle du pont nasal (13), notamment de l'échancrure du pont nasal, est déterminée à partir de l'ensemble de données numériques, dans lequel un plan de référence supplémentaire (17) qui s'étend parallèlement au plan coronal (15) à travers le pont nasal, notamment à travers l'échancrure du pont nasal, est déterminé, dans lequel les positions coronales des yeux (10) sont également déterminées par rapport au plan de référence supplémentaire (17) de sorte que les positions coronales des yeux (10) sont déterminées plus précisément.

4. Procédé selon la revendication 2 ou 3, dans lequel la position coronale est déterminée par rapport aux positions (12-1, 12-2) du tragus de chaque oreille (12), par rapport à la seconde ligne de connexion et/ou par rapport au pont nasal (13).

5. Procédé selon l'une quelconque des revendications précédentes, dans lequel un procédé de détection de pupille mis en oeuvre par ordinateur est utilisé pour déterminer automatiquement les positions de centre (10-1, 10-2) des yeux (10) dans l'ensemble de données numériques.

6. Procédé selon l'une quelconque des revendications précédentes, dans lequel un procédé de détection de bord mis en oeuvre par ordinateur est utilisé pour déterminer automatiquement les positions de bord (11-1, 11-2).

7. Procédé selon l'une quelconque des revendications précédentes, dans lequel les informations concernant la surface tridimensionnelle sont représentées sous forme de maille polygonale à faces-sommets dans l'ensemble de données numériques.

8. Procédé selon l'une quelconque des revendications précédentes, dans lequel l'ensemble de données numériques comprend des informations concernant une couleur ou une texture d'échelle de gris de la surface tridimensionnelle, dans lequel lesdites informations sont associées à la surface tridimensionnelle.

9. Procédé selon les revendications 7 et 8, dans lequel les informations concernant la couleur ou l'échelle de gris sont associées à la maille polygonale sous forme d'une carte de texture mappant les informations concernant la couleur ou l'échelle de gris aux faces et sommets de la maille polygonale.

10. Procédé selon l'une quelconque des revendications 6 à 9, dans lequel le bord orbital latéral antérieur est marqué avec un marqueur ayant une couleur différente d'un teint de la personne avant que l'ensemble de données numériques ne soit acquis, dans lequel les positions de bord (11-1, 11-2) sont déterminées par le procédé de détection de bord en recherchant la couleur du marqueur dans l'ensemble de données numériques.

11. Procédé selon l'une quelconque des revendications 6 à 10, dans lequel les positions de centre (10-1, 10-2) des pupilles sont d'abord déterminées, puis les positions de bord (11-1, 11-2) sont déterminées par le procédé de détection de bord, dans lequel le procédé de détection de bord limite sa recherche de positions de bord à des portions du visage qui sont situées latéralement des positions de centre déterminées (10-1, 10-2) des pupilles.

12. Procédé selon l'une quelconque des revendications précédentes, dans lequel le procédé de détection de pupille exécute les étapes suivantes :

- déterminer une portion de l'ensemble de données numériques qui comprend les yeux (10),
- filtrer les données numériques représentant ladite portion avec un filtre passebas,
- déterminer la position de centre (10-1, 10-2) des pupilles à partir des données numériques filtrées.

13. Procédé selon l'une quelconque des revendications précédentes, dans lequel le dispositif de balayage de surface tridimensionnelle portable est compris dans un dispositif mobile, tel qu'un smartphone ou une tablette.

14. Programme informatique comprenant du code de programme informatique qui, lors de son exécution sur un ordinateur, amène l'ordinateur à exécuter au moins les étapes mises en oeuvre par ordinateur suivantes du procédé selon l'une quelconque des revendications précédentes :

- lire à partir d'une mémoire de l'ordinateur l'ensemble de données numériques,
- déterminer les positions de centre (10-1, 10-2) des pupilles avec le procédé de détection de pupille,
- déterminer les positions de bord (11-1, 11-2) avec le procédé de détection de bord,
- déterminer une ligne de connexion (11-3) entre les deux positions de bord (11-1, 11-2),
- déterminer pour chaque position de centre (10-1, 10-2) des pupilles une distance à la ligne de connexion (11-3), lesdites distances correspondant à la position coronale de chaque oeil (10) par rapport au plan coronal (15) de la tête ;
- afficher des informations concernant les positions coronales déterminées sur un affichage.

15. Procédé informatique selon la revendication 14, dans lequel le programme informatique exécute en outre les étapes consistant à :

- comparer les positions coronales déterminées à des positions coronales qui ont été déterminées lors de jours précédents à partir de la même personne,
- afficher une représentation graphique fournissant des informations concernant un écart des positions coronales déterminées et des positions coronales qui ont été déterminées lors de jours précédents.

Fig. 1

Fig. 2

Surface scan comprising of a
portion of the face comprising the
eyes and ears, generating a data
set with three-dimensional
surface and texture data.

101

Determining the center of the
pupils of each eye in three
dimensions from the three-
dimensional surface and texture
data

102

Determining the orbital rim
position in three-dimensions from
the three-dimensional surface
data

103

Determining a connecting line
between the rim positions

104

Determining the distance of each
center positon of the pupils to the
connecting line and assigning
said distance to the coronal
positon of the respective eye

105

Fig. 3

A)

1

15

B)

12-1    11-1    10-1    13    10-2    11-2    12-2

f1

11-3    12-3

Fig. 3

C)

D)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020110121 A1

- US 2012010533 A1

**Non-patent literature cited in the description**

- **BENZ et al.** Optical 3d-metrology for medical applications. *Proceedings of ICMP 2005 and BMT, Berlin,* 2005, 64-65

- **VIOLA ; JONES.** Rapid object detection using a boosted cascade of simple features. *International Journal of Computer Vision,* 2001